# EUROPEAN PATENT SPECIFICATION

(11) **EP 1 296 733 B1**
(45) Date of publication and mention of the grant of the patent: **29.11.2006**
(21) Application number: 01943667.4
(22) Date of filing: 28.06.2001
(51) Int. Cl.: A61M 15/00, A61M 15/08, B05B 11/00

(54) **DISPENSING APPARATUS**
SPENDER FÜR MEDIEN
DISPOSITIF DISTRIBUTEUR

(30) Priority: 06.07.2000 GB 0016670; 21.08.2000 GB 0020600
(43) Date of publication of application: 02.04.2003
(73) Proprietor: Bespak plc, King's Lynn Norfolk PE30 2JJ (GB)
(72) Inventor: POLLET, Hilde, King's Lynn, Norfolk PE30 5DA (GB); BEKEN, Sally, Rowlands Castle, Hampshire PO9 6BB (GB)
(74) Representative: Alexander, Thomas Bruce
(86) International application number: PCT/GB2001/002897
(87) International publication number: WO 2002/004056

(56) References cited:
- EP-A- 0 808 635
- EP-A- 0 906 765
- WO-A-00/16835
- WO-A-98/51360
- WO-A-98/55168
- WO-A-99/49923
- DE-A- 19 700 838
- US-A- 4 417 890
- US-A- 5 884 820
- US-A- 6 039 042
- US-A- 6 120 481

## Description

This invention relates to dispensing apparatus. In particular, the invention relates to dispensing apparatus for pharmaceutical products and preparations having improved tactile and hygiene features.

Many types of dispensing apparatus are known for use in dispensing pharmaceutical products and preparations. These include oral actuators, nasal actuators, spacers, ophthalmic dispensers, dermal applicators (transdermal or hypodermic), needleless injectors, aural dispensers, vaginal dispensers and the like.

Oral actuators typically comprise a container in which the medicament is stored. Such containers include a dispensing container, in which the medicament is stored as a liquid medium. The dispensing container may be 'pressurised' wherein the medicament is dispensed using a volatile propellant or 'unpressurised' wherein the medicament is dispensed using a pump or other non-liquified gas system. Alternatively, the medicament can be dispensed from frangibly sealed containers, in which the medicament is stored as a dry powder and dispensed using an air stream. Variants of such oral actuators include metered dose inhalers, pressurised metered dose inhalers, dry powder inhalers, breath actuated inhalers and breath co-ordinated inhalers.

Nasal actuators typically comprise a vial or other container for storage of the medicament. Dispensation of the medicament via an outlet nozzle is achieved by use of a volatile propellant, driven by an air stream or by means of a pressure being applied to the liquid medicament via a drive means. Alternatively, dispensation may be gravity driven, as in the case of 'droppers' for nose drop preparations.

Spacers are used in conjunction with another dispensing apparatus to provide a chamber or other generally enclosed space in which the velocity of dispensed medicament particles may be slowed before they are administered to a user, either orally or nasally.

Ophthalmic dispensers include 'droppers' for eye drop preparations, eye-baths for bathing of the eye with a medicament 'wash' and dispensing containers (either pressurised or unpressurised) for dispensing medicaments as low velocity mists towards the eye.

Dermal applicators include hypodermic syringes, catheters and other such devices which physically penetrate the skin boundary layer.

Needleless injectors, unlike hypodermic syringes, are used for dispensing medicaments subcutaneously but without any portion of the injector penetrating the skin boundary layer. Typically such injectors work by driving the medicament at high velocities across the skin boundary using a compressed medium such as compressed air to provide the driving force.

Aural dispensers include 'droppers' for ear drop preparations and other such applicators for applying liquids, creams or the like into the ear.

Vaginal dispensers include devices for applying liquids, creams and like preparations into the vagina.

A common feature of all of the above described apparatus is that an outlet is provided through which the medicament is dispensed. The outlet may be a mouthpiece in the case of, for example, oral actuators and spacers or a generally conically-shaped tip in the case of, for example, nasal, aural, dermal or vaginal actuators. The outlet is typically contacted by the user during use. Therefore, it is important that the surfaces of the outlet are kept free from dirt, dust and other contaminants. In addition, it is advantageous to provide a means for sealing the outlet of the apparatus to prevent the ingress of dirt, dust or other contaminants into the body of the apparatus.

One problem with such dispensing apparatus is that the material of the apparatus can cause discomfort where it comes into contact with the user during dispensation. For example, known polymeric plastics such as polypropylene and high density polyethylene can feel 'cold' to the touch and may become abraided during use, forming sharpened edges or corners which may cause pain or injury to a user. For example, the mouthpiece or sealing cap of an oral actuator may scratch or cut the tongue, lips or face of a user if any sharpened edges or corners are present. Likewise, the tip of a nasal actuator may cut or scratch the nasal lining of a user if it becomes roughened.

Another problem with such dispensing apparatus is that they can be difficult to grasp and handle especially by the young or infirm. Typical polymeric plastics used for the housings of such dispensing apparatus tend to be reasonably slippery especially when wet. In addition the apparatus can be difficult to grasp and operate especially one-handed.

EP0808635 is considered to be the closest prior art and discloses an apparatus for dispensing a medicament, comprising a housing defining an outlet through which, in use, medicament is dispensed, a removable cap engageable with the outlet to close the outlet, and a strap connecting the cap to the housing whereby the cap is still attached to the housing when disengaged from the outlet. The present invention is characterised from the prior art in that the strap is formed from a thermoplastic elastomer having sufficient elasticity to accommodate the engagement and disengagement of the cap with the outlet.

Embodiments of the present invention will now be described, by way of example only, with reference to the accompanying drawings, in which:
Figure 1 is perspective view of a first dispensing apparatus;
Figure 2 is perspective view of a second dispensing apparatus;
Figure 3 is perspective view of a third dispensing apparatus;
Figure 4 is a perspective view of a fourth dispensing apparatus, this being an embodiment of apparatus according to the present invention, having an integrally formed strap attached to a side wall and with the cap disengaged from the mouthpiece.
Figure 5a is a cross-sectional schematic view of a fifth dispensing apparatus, this being a further embodiment of apparatus according to the present invention, having an integrally formed strap attached to an end wall and the cap being in the engaged position on the mouthpiece;
Figure 5b is a cross-sectional schematic view of the apparatus of Figure 5a showing the cap in co-axial alignment with the mouthpiece immediately prior to movement of the cap into the engaged position on the mouthpiece;
Figure 5c is a cross-sectional schematic view of the apparatus of Figure 5a showing the cap in an unengaged position, for example, during actuation of the apparatus;
Figure 6a is side view of a sixth dispensing apparatus, this being an embodiment of dispensing apparatus according to the present invention;
Figure 6b is an end view of the apparatus of Figure 6a;
Figure 7 is a sectional view of a seventh dispensing apparatus, this being an embodiment of dispensing apparatus according to the present invention;
Figure 8 is a sectional view of an eighth dispensing apparatus, this being an embodiment of dispensing apparatus according to the present invention; and
Figure 9 is an enlarged sectional view of the outlet of the apparatus of Figure 8.

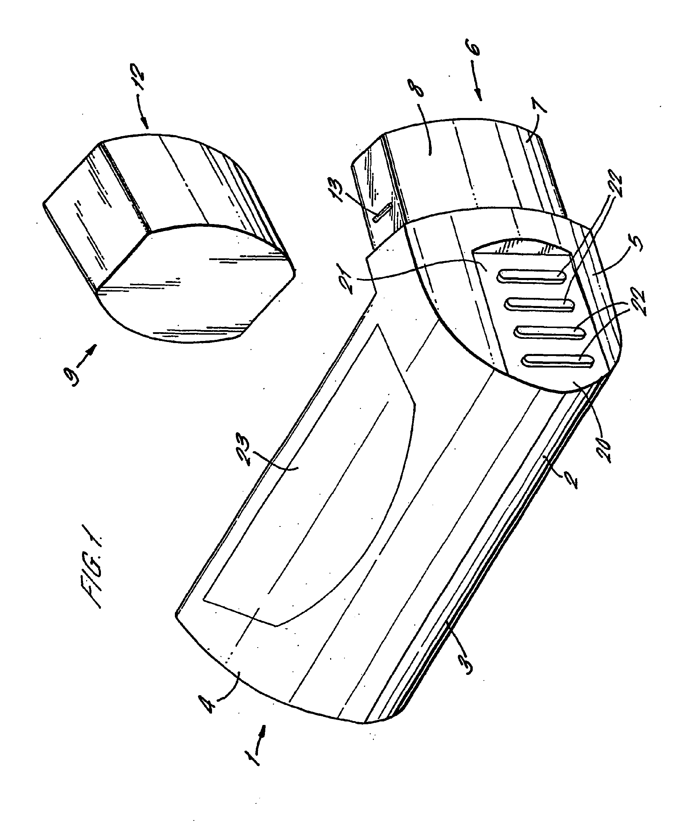

Figures 1 through 6b show six variants of dispensing apparatus. The dispensing apparatus have in common an apparatus 1 comprising a housing 2 consisting of a tubular body 3 having a tubular side wall and an open end 4. The body 3 is closed at its opposite end by an end wall 5 and a tubular mouthpiece 6 projects laterally of the body at a location immediately adjacent the end wall 5. The mouthpiece 6 has a tubular lip portion 7 having an external surface 8 which in use is presented to the lips of a user wishing to inhale orally via the mouthpiece an aerosol spray generated from a pressurised dispensing container (not shown) normally received within the body 3.

The apparatus 1 further comprises a cap 9. The cap 9 is a sliding fit onto the lip portion 7 such that an internal surface 12 of the cap totally overlays the external surface 8 of the lip portion when the cap is moved into an engaged position in which it is engaged with the mouthpiece 6.

The lip portion 7 and the cap 9 are provided with co-operating snap fit connectors which include a detent 13 projecting from the lip portion in co-operating relationship with a groove (not shown) formed in the internal surface 12 of the cap 9.

In known apparatus the housing 2 and cap 9 are typically formed from a plastics material such as polypropylene or high density polyethylene (HDPE).

The apparatus is formed as a co-moulding of HDPE, polypropylene or similar together with a thermoplastic elastomer to form one or more surface features on the apparatus 1 to improve the apparatus' hygiene and/or tactile features.

In a first step of the co-moulding the housing 2 is moulded from polypropylene, HDPE or a similar material. In a second step the thermoplastic elastomer components of the apparatus are moulded intimately with the housing 2. The thermoplastic elastomer components are retained on the housing 2 by a mixture of physical and chemical bonding. In addition, and if required, additional fixing means may be used to provide a superior bond, e.g. welding, mechanical fasteners, glue etc.

Preferably both steps of the co-moulding process are carried out in the same mould tool. Alternatively, the moulded housing 2 may be moved to a different mould tool for the second step. The two mould steps could be reversed in order. The thermoplastic elastomer used can be any of Santoprene, Pebax, Vitaprene, Hytrel or the like. Various thermoplastic elastomer components may be advantageously moulded onto the housing 2 as described below.

In the dispensing apparatus, shown in Figure 1, a pad 20 of a thermoplastic elastomer is formed on end wall 5. Preferably, the pad 20 is provided with ridges 22 or other surface protrusions or indentations. These serve to improve the grip of the user's finger or thumb when holding the apparatus 1. The pad 20 is preferably "keyed" into a recessed "key-way" 21 in the end wall 5 of the tubular body 3. The "key" and "key-way" arrangement provides an improved connection between the thermoplastic elastomer and the polypropylene or HDPE material. Alternatively, the pad 20 may be simply bonded to the surface of the end wall 5 without the use of a "key-way". The housing 2 may be provided with a re-entrant feature through which the pad 20 is moulded to provided for improved attachment of the pad 20 to the housing 2.

One or more portions 23 of the tubular body 3 are provided with a portion of thermoplastic elastomer. The thermoplastic elastomer portions 23 provide for an improved grip on the sides of the tubular body 3. The portions 23 may be bonded on the surface of the tubular body 3 or "keyed" into recesses formed in the surface. Writing and/or pictures may be formed in the portions 23 either as "cutout" areas or as indentations or protrusions. In this way information such as dosage instructions, product warnings and brand logos may be provided on the housing 2. Raised writing or braille notation may be provided for the visually impaired. Advantageously, the writing and/or pictures, being an intimate part of the apparatus 1, are resistant to being removed or obliterated over time. This is especially important for dosage instructions and warning notices which have previously tended to be provided on sticky labels or similar, which may easily be removed.

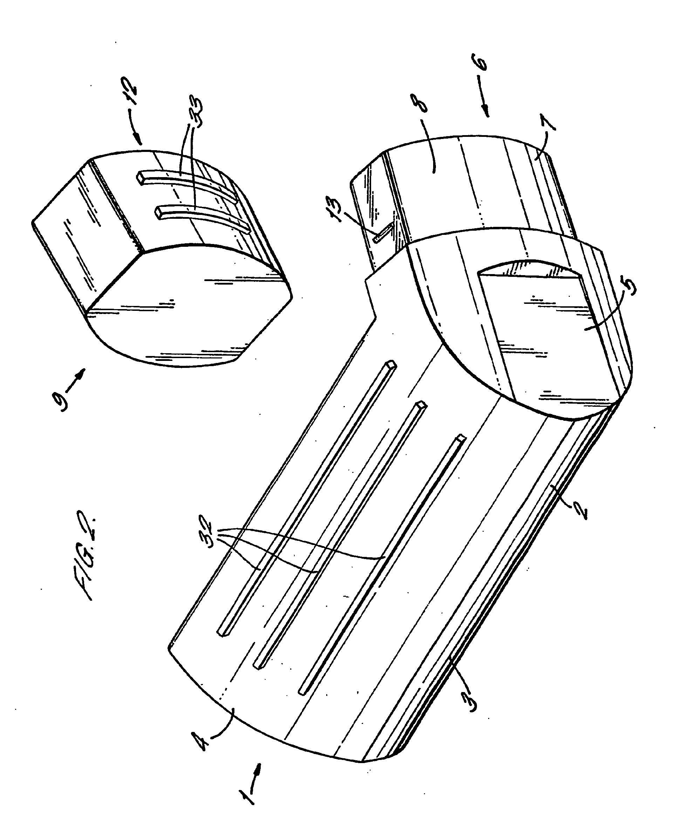

In the dispensing apparatus, shown in Figure 2, a series of longitudinally orientated ridges 32 are formed on the sides of tubular body 3. The ridges are formed from a co-moulded thermoplastic elastomer of the type, and in the manner, described above. Ridges 33 may also be provided on cap 9. Preferably the ridges 33 are orientated laterally so as to improve the finger-grip of a user pulling or pushing on the cap 9. The ridges 32, 33 may be "keyed" into the surface or bonded onto the surface.

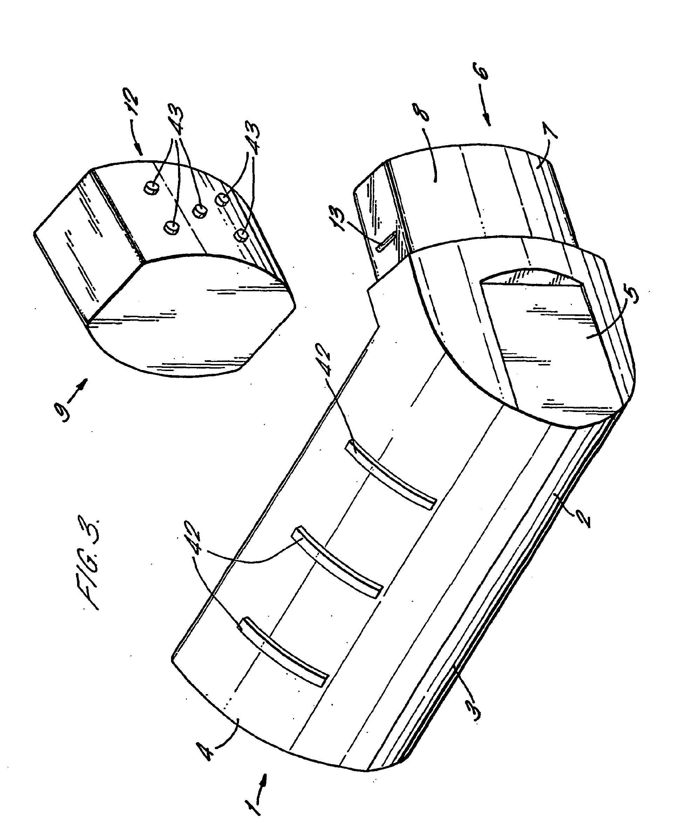

The third dispensing apparatus comprises a similar arrangement, shown in Figure 3, except that the ridges 42 are diagonally orientated and that raised dots 43 are provided on the cap 9.

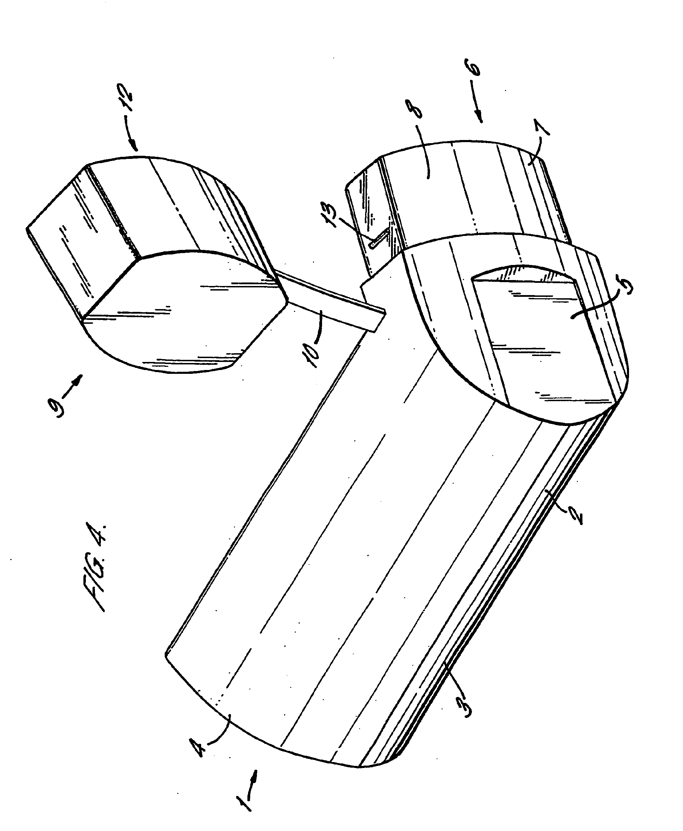

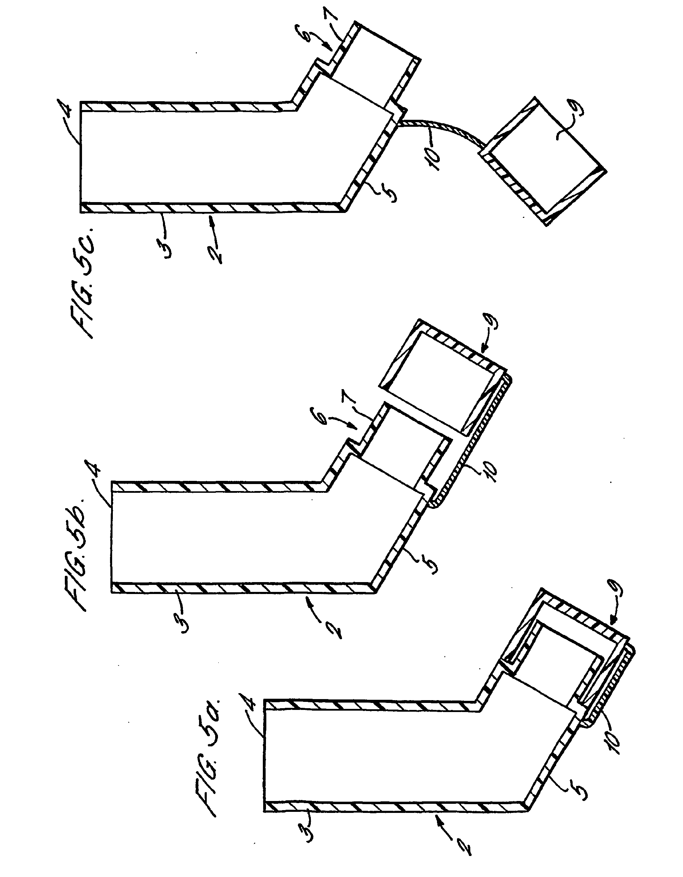

Figure 4 shows a fourth dispensing apparatus, this being an embodiment of the present invention, whilst Figures 5a to 5c show a fifth dispensing apparatus, also an embodiment of the present invention.

Referring to Figure 4, the cap 9 is connected to the body 3 by a strap 10 formed of a thermoplastic elastomer material such as Santoprene (RTM), Pebax (RMT), Vitaprene (RTM), Hytrel (RTM) or the like. The strap 10 is joined to the cap 9 and housing 2 by mechanical fasteners, glue, heated or ultrasonic welds, or a combination of these means.

In the embodiment of a dispensing apparatus shown in Figure 4, the cap 9 is attached to a side of the tubular body 3. In the fifth embodiment, shown in Figures 5a to 5c, the strap 10 is attached to the end wall 5. In other respects the fourth and fifth embodiments are identical. As such, the engagement and disengagement of the cap will now be described with reference to the fifth embodiment only.

Advantageously, the inherent elasticity of the thermoplastic elastomer material allows the strap 10 to stretch and extend in length when the cap 9 is pulled in a direction away from the tubular body 3.

The cap 9 is a sliding fit onto the lip portion 7 such that an internal surface 12 of the cap totally overlays the external surface 8 of the lip portion when the cap is moved into an engaged position as shown in Figure 5a in which it is engaged with the mouthpiece 6.

The lip portion 7 and the cap 9 are provided with co-operating snap-fit connectors which include a detent 13 projecting from the lip portion in co-operating relationship with a groove (not shown) formed in the internal surface 12 of the cap 9.

As shown in Figure 5c, the cap is movable when disengaged from the mouthpiece 8 into a position in which it lies at a location which is offset from the axial extent of the body 3 and from the axial extent of the mouthpiece 6 by a distance determined by the length of the strap 10. Due to the relative flexibility of the strap 10 the cap tends to 'fall away' from the mouthpiece 6 when it is disengaged which greatly improves access to the mouthpiece 6.

In this position, the user is able to grip the housing without interference from the presence of the cap 9 and strap 10, the user typically resting a thumb against the end wall 5 and an index finger around the barrel shaped body 3.

In order to move the cap 9 from this position of Figure 5c into the engaged position of Figure 5a it is necessary to move the cap 9 away from the body 3 into co-axial alignment with the mouthpiece 6 at a position in which the cap 9 extends beyond the axial extent of the lip portion 7, as shown in Figure 5b. During this movement the strap 10 is stretched. The maximum extended length of the strap 10 must correspond at least to this configuration. Movement of the cap into the engaged position then proceeds by pushing the cap 9 towards the body 3 in sliding relationship relative to the lip portion 7 until the detent 13 effects a snap fit connection and the cap rests in the fully engaged position shown in Figure 5a in which the entire external surface 8 of the lip portion 7 is overlaid.

In the engaged position of Figure 5a it is seen that the strap 10 has elastically recovered to its original, unstretched length, such that the strap 10 is held close to the body 3 and does not extend away from the body 3 in a loop formation. As a result the risk of catching the strap 10 on an object and accidentally dislodging the cap 10 is greatly reduced. Also the overall dimensions of the apparatus in the storage condition are reduced.

Cap 9 may be formed from a plastics material such as polypropylene. Advantageously, according to a further aspect of the present invention, the cap 9 may also be formed from a thermoplastic elastomer. The cap 9 and strap 10 may then be formed as a unitary body by means of a moulding process. The inherent flexibility of the thermoplastic elastomer allows the cap 9 to be distorted or crushed and yet return to its original shape. This is advantageous in that it both simplifies removal and engagement of the cap 9 with the mouthpiece 8 as the cap 9 can distort somewhat but also in that the cap does not risk scratching the face of the user during use.

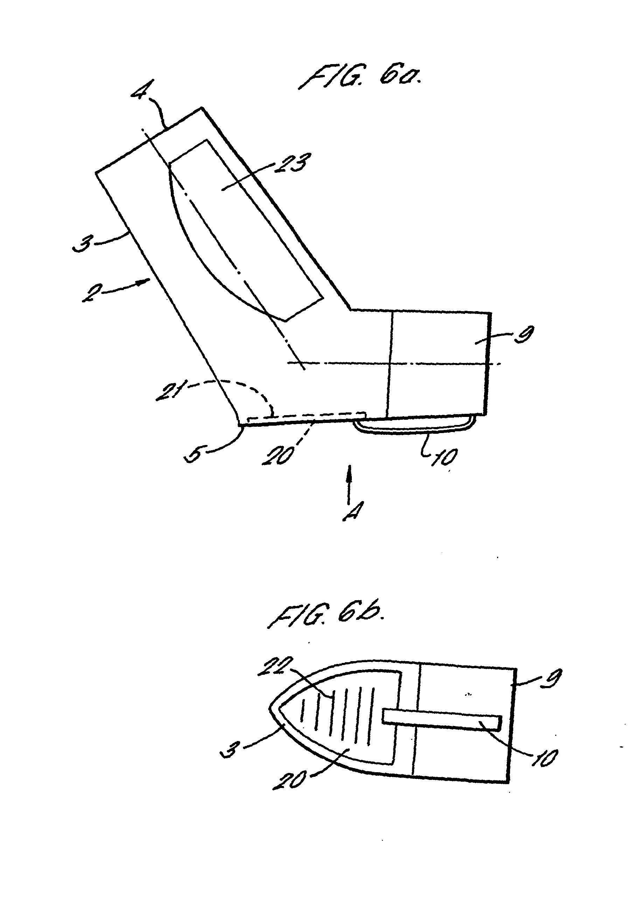

Figures 6a and 6b show a sixth dispensing apparatus, this being an embodiment of the present invention. Similar components to those shown in the previous embodiments have been referenced with the same reference numerals and will not be described here in any more detail. Strap 10 is connected to the cap 9 as in the fifth embodiment. At the other end of the strap 9 is formed a pad 20 of a thermoplastic elastomer material, such as Santoprene Pebax, Vitaprene, Hytrel or the like. The pad 20 may be connected to the strap 10 by any of the methods described above in connection with the previous embodiments. However, preferably the strap 10 and pad 20 are formed as a unitary body by a moulding process. In the case where the strap 10, cap 9 and pad 20 are all formed of a thermoplastic elastomer, preferably they are formed as a single unitary body by means of a moulding process.

The pad 20 serves as a "key" which is received in a recessed "key-way" 21 in the end wall 5 of the tubular body 3. The pad 20 is fixedly attached to the recessed "key-way" 21 by any of the methods described above in the first embodiment, e.g. welding, mechanical fasteners, glue etc. The "key" and "key-way" arrangement provides an improved connection between the thermoplastic elastomer and the polypropylene materials. Alternatively the pad 20 may be simply bonded to the surface of the end wall 5 without the use of a key-way.

Advantageously, where the cap 9, strap 10 and pad 20 are all formed from a thermoplastic elastomer the apparatus may be formed as a two-step moulding. In the first step the housing 2 is moulded from a polypropylene or similar material and in the second step the cap 9, strap 10 and pad 20 are moulded from a thermoplastic elastomer directly into position on the housing 2, with the pad 20 being retained on the housing 2 by a mixture of physical and chemical bonding. Preferably both steps of the moulding process are carried out in the same mould tool. Alternatively, the moulded housing 2 may be moved to a different mould tool for the second step. The two mould steps could be reversed in order.

Optionally the housing 2 is provided with a re-entrant feature through which the pad 20 is moulded to provided for improved attachment of the pad 20 to the housing 2.

Preferably the pad 20 is provided with ridges 22 or other surface protrusions or indentations. These serve to improve the grip of the user's finger or thumb when holding the apparatus.

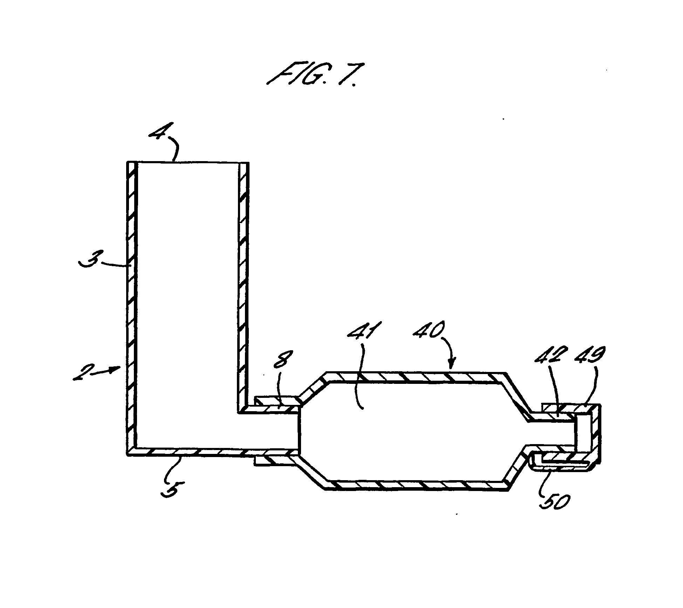

Figure 7 shows a seventh dispensing apparatus, this being an embodiment of dispensing apparatus in the form of a spacer 40 attached to an oral actuator of the type shown in Figures 1 to 6b.

The spacer 40 attaches to the mouthpiece 8 of the oral actuator and provides a chamber 41 in which the dispensed medicament particles slow before inhalation. A mouthpiece 42 generally opposite the mouthpiece 8 of the oral actuator is provided with a cap 49 which may be attached to the spacer body by means of a strap 50. The structure, materials and operation of the strap and cap arrangement are the same as described above. Of course, the strap and cap arrangement of the present invention may be applied to other types of spacer or where the spacer is attached to other types of actuator.

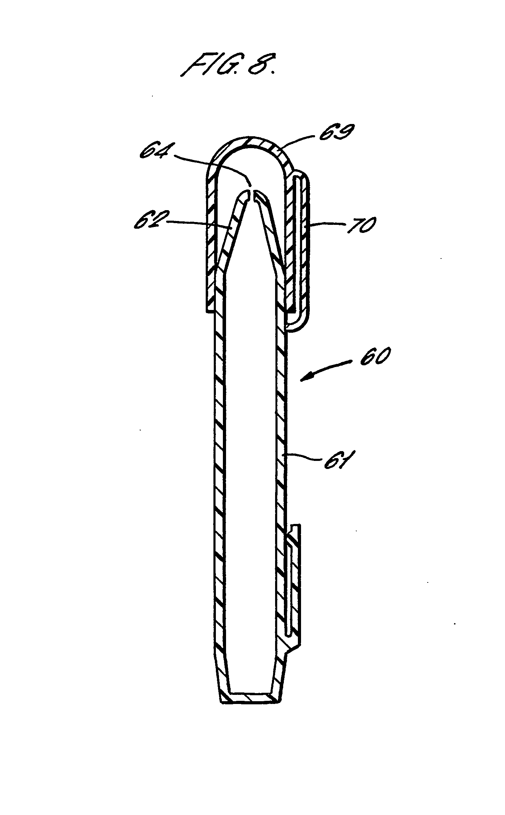

Figure 8 shows an eighth dispensing apparatus, this being an embodiment of the present invention comprising a nasal actuator 60 having a body 61, tip 62 and cap 69. The tip 62 defines an outlet 64 through which medicament is dispensed. The nasal actuator 60 is provided with a cap 69 which may be attached to the spacer body by means of a strap 70.

The structure, materials and operation of the strap and cap arrangement are the same as described above.

The spacer 40 and nasal actuator 60 may comprise any of the thermoplastic elastomer components described with reference to the oral actuators of Figures 1 to 6b. For example, the spacer 40 may be provided with ribs to aid the user's grip when attaching and detaching the spacer 40.

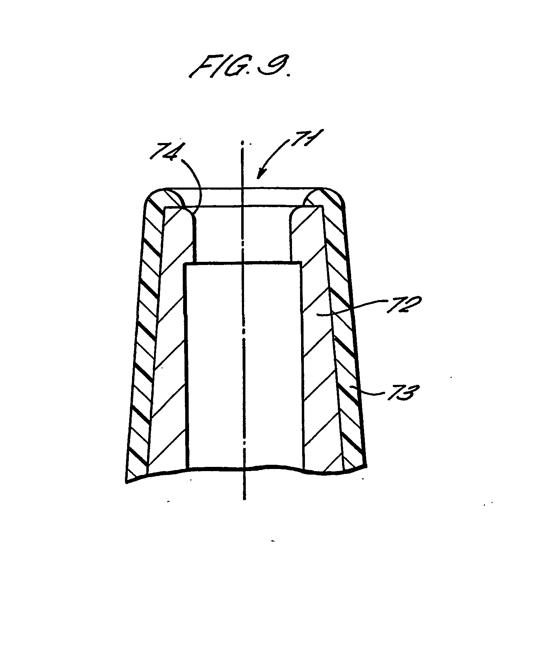

Figure 9 shows the use of a thermoplastic elastomer co-moulding 73 for forming the outlet tip of a nasal actuator. A rigid core 72 of polymeric plastics or metal which defines an outlet orifice 71 is overlaid by a layer 73 of thermoplastic elastomer of the type described above. Preferably, the thermoplastic elastomer extends over the entire surface contacted by the user's nose whilst leaving the internal surface 74 of the outlet orifice 71 free of thermoplastic elastomer. In this way the spray pattern which depends on the orifice geometry is not adversely affected. Advantageously, the thermoplastic elastomer coating 73 provides a comfortable, soft and hygienic contact surface for the user. In addition the material feels 'warmer' to the touch which has been found to be a desirably characteristic. The thermoplastic elastomer is formed as a co-moulding as described above.

The concept of the present invention may be applied to other types of dispensing apparatus such as ophthalmic dispensers, dermal applicators (transdermal or hypodermic), needleless injectors, aural dispensers and vaginal dispensers. Any of the thermoplastic elastomer components described with reference to the oral actuators of Figures 1 to 6b and nasal actuator of Figure 8 may be incorporated in such dispensing apparatus as appropriate. For example a thermoplastic elastomer-coated tip may be provided on an aural dropper for use with ear drops or thermoplastic elastomer ribs may be provided on the housing of a needleless actuator.

## Claims

1. Apparatus (1) for dispensing a medicament, comprising a housing (2) defining an outlet (6) through which, in use, medicament is dispensed, a removable cap (9) engageable with the outlet (6) to close the outlet (6), and a strap (10) connecting the cap (9) to the housing whereby the cap (9) is still attached to the housing (2) when disengaged from the outlet (6), **characterised in that** the strap (10) is formed from a thermoplastic elastomer having sufficient elasticity to accommodate the engagement and disengagement of the cap with the outlet.

2. Apparatus as claimed in claim 1 wherein the length of the strap (10) is such that the strap (10) lies in close proximity with the housing (2) when the cap (9) is engaged with the outlet (6).

3. Apparatus as claimed in claim 1 or claim 2 wherein the cap (9) is formed from a thermoplastic elastomer.

4. Apparatus as claimed in claim 3 wherein the cap (9) and strap (10) are formed as a unitary body.

5. Apparatus as claimed in any of claims 1 to 4 wherein a pad (20) of thermoplastic elastomer material is formed at one end of the strap (10), the pad (20) being fixedly attached to the housing (2) to form a non-slip surface.

6. Apparatus as claimed in claim 5 wherein the pad (20), strap (10) and cap (9) are formed as a unitary body.

7. Apparatus as claimed in claim 6 wherein one of the housing (2) or the unitary body comprising the pad (20), strap (10) and cap (9) is formed in a first moulding step and the other of the housing or the unitary body comprising the pad (20), strap (10) and cap (9) is formed in a second moulding step.

8. Apparatus as claimed in any of claims 1 to 7 wherein one or more portions of the housing (2) are coated with thermoplastic elastomer to form a non-slip surface.

9. Apparatus as claimed in any of claims 1 to 8 wherein movement of the cap (9) into and out of engagement with the outlet (6) is accompanied by co-axial relative movement of the cap (9) and the outlet (6).

10. Apparatus as claimed in any of claims 1 to 9 wherein the apparatus comprises a pressurised dispensing container operable to dispense an aerosol spray.

11. Apparatus as claimed in any of claims 1 to 9 wherein the apparatus comprises an inhalator operable to dispense a powdered medicament.

12. Apparatus as claimed in any of claims 1 to 9 wherein the apparatus comprises a nasal actuator (60) .

13. Apparatus as claimed in any of claims 1 to 9 wherein the apparatus is a spacer (40).

## Patentansprüche

1. Vorrichtung (1) zum Abgeben eines Medikaments, umfassend ein Gehäuse (2), das einen Auslass (6) definiert, durch den bei Gebrauch Medikament abgegeben wird, eine abnehmbare Kappe (9), die mit dem Auslass (6) zum Schließen des Auslasses (6) in Eingriff bringbar ist, und ein Band (10), das die Kappe (9) mit dem Gehäuse verbindet, wodurch die Kappe (9) immer noch an dem Gehäuse (2) angebracht ist, wenn sie außer Eingriff mit dem Auslass (6) gebracht ist, **dadurch gekennzeichnet, dass** das Band (10) aus einem thermoplastischen Elastomer gebildet ist, welches eine ausreichende Elastizität aufweist, um das Bringen der Kappe in Eingriff und außer Eingriff mit dem Auslass aufzunehmen.

2. Vorrichtung nach Anspruch 1, wobei die Länge des Bandes (10) derart ist, dass sich das Band (10) in enger Nähe zu dem Gehäuse (2) befindet, wenn die Kappe (9) mit dem Auslass (6) in Eingriff steht.

3. Vorrichtung nach Anspruch 1 oder Anspruch 2, wobei die Kappe (9) aus einem thermoplastischen Elastomer gebildet ist.

4. Vorrichtung nach Anspruch 3, wobei die Kappe (9) und das Band (10) als ein einheitlicher Körper ausgebildet sind.

5. Vorrichtung nach einem beliebigen der Ansprüche 1 bis 4, wobei ein Polster (20) aus einem thermoplastischen Elastomermaterial an einem Ende des Bandes (10) ausgebildet ist, wobei das Polster (20) fest an dem Gehäuse (2) zur Bildung einer rutschfesten Oberfläche angebracht ist.

6. Vorrichtung nach Anspruch 5, wobei das Polster (20), das Band (10) und die Kappe (9) als ein einheitlicher Körper ausgebildet sind.

7. Vorrichtung nach Anspruch 6, wobei eines aus dem Gehäuse (2) oder dem einheitlichen Körper, umfassend das Polster (20), das Band (10) und die Kappe (9), in einem ersten Formungsschritt gebildet ist und das andere aus dem Gehäuse und dem einheitlichen Körper, umfassend das Polster (20), das Band (10) und die Kappe (9), in einem zweiten Formungsschritt gebildet ist.

8. Vorrichtung nach einem beliebigen der Ansprüche 1 bis 7, wobei einer oder mehrere Abschnitte des Gehäuses (2) mit thermoplastischem Elastomer beschichtet sind, um eine rutschfeste Oberfläche zu bilden.

9. Vorrichtung nach einem beliebigen der Ansprüche 1 bis 8, wobei die Bewegung der Kappe (9) in und außer Eingriff mit dem Auslass (6) durch eine koaxiale Relativbewegung der Kappe (9) und des Auslasses (6) begleitet ist.

10. Vorrichtung nach einem beliebigen der Ansprüche 1 bis 9, wobei die Vorrichtung einen unter Druck stehenden Spendebehälter umfasst, der zum Abgeben eines Aerosolsprays betätigbar ist.

11. Vorrichtung nach einem beliebigen der Ansprüche 1 bis 9, wobei die Vorrichtung einen Inhalator umfasst, der zur Abgabe eines pulverförmigen Medikaments bedienbar ist.

12. Vorrichtung nach einem beliebigen der Ansprüche 1 bis 9, wobei die Vorrichtung einen Nasalaktuator (60) umfasst.

13. Vorrichtung nach einem beliebigen der Ansprüche 1 bis 9, wobei die Vorrichtung ein Zwischenstück (40) ist.

## Revendications

1. Appareil (1) pour distribuer un médicament, comprenant un boîtier (2) définissant une sortie (6) à travers laquelle, en utilisation, un médicament est distribué, un capuchon amovible (9) pouvant engager avec la sortie (6) pour fermer la sortie (6), et une attache (10) reliant le capuchon (9) au boîtier, ce par quoi le capuchon (9) demeure assujetti au boîtier (2) lorsqu'il est dégagé de la sortie (6), **caractérisé en ce que** l'attache (10) est formée d'un élastomère thermoplastique ayant une élasticité suffisante pour permettre l'engagement du capuchon avec la sortie et son dégagement de celle-ci.

2. Appareil selon la revendication 1, dans lequel la longueur de l'attache (10) est telle que l'attache (10) se trouve à proximité immédiate du boîtier (2) lorsque le capuchon (9) est engagé avec la sortie (6).

3. Appareil selon la revendication 1 ou la revendication 2, dans lequel le capuchon (9) est formé d'un élastomère thermoplastique.

4. Appareil selon la revendication 3, dans lequel le capuchon (9) et l'attache (10) sont formés comme un corps unitaire.

5. Appareil selon l'une quelconque des revendications 1 à 4, dans lequel un patin (20) de matériau élastomère thermoplastique est formé au niveau d'une extrémité de l'attache (10), le patin (20) étant assujetti de façon fixe au boîtier (2) pour former une surface non glissante.

6. Appareil selon la revendication 5, dans lequel le patin (20), l'attache (10) et le capuchon (9) sont formés comme un corps unitaire.

7. Appareil selon la revendication 6, dans lequel l'un du boîtier (2) ou du corps unitaire comprenant le patin (20), l'attache (10) et le capuchon (9) est formé lors d'une première étape de moulage et l'autre du boîtier ou du corps unitaire comprenant le patin (20), l'attache (10) et le capuchon (9) est formé lors d'une seconde étape de moulage.

8. Appareil selon l'une quelconque des revendications 1 à 7, dans lequel une ou plusieurs parties du boîtier (2) sont revêtues d'élastomère thermoplastique pour former une surface non glissante.

9. Appareil selon l'une quelconque des revendications 1 à 8, dans lequel le déplacement d'engagement du capuchon (9) avec la sortie (6) et de son dégagement de celle-ci s'accompagne d'un déplacement coaxial relatif du capuchon (9) et de la sortie (6).

10. Appareil selon l'une quelconque des revendications 1 à 9, dans lequel l'appareil comprend un conteneur de distribution pressurisé pouvant opérer pour distribuer une pulvérisation d'aérosol.

11. Appareil selon l'une quelconque des revendications 1 à 9, dans lequel l'appareil comprend un inhalateur pouvant opérer pour distribuer un médicament sous forme de poudre.

12. Appareil selon l'une quelconque des revendications 1 à 9, dans lequel l'appareil comprend un actionneur nasal (60).

13. Appareil selon l'une quelconque des revendications 1 à 9, dans lequel l'appareil est un écarteur (40).
